# FASCICULE DE BREVET EUROPEEN

(11) **EP 1 379 623 B1**
(45) Date de publication et mention de la délivrance du brevet: **04.03.2009**
(21) Numéro de dépôt: 01976355.6
(22) Date de dépôt: 03.10.2001
(51) Int. Cl.: C12N 1/20, C12Q 1/04, C02F 3/02, C02F 3/34

(54) **BACTERIES UTILISABLES POUR OXYDER DE L'ARSENIC, PROCEDE POUR LEUR SELECTION ET LEURS APPLICATIONS POUR TRAITER DES MILIEUX RENFERMANT DE L'ARSENIC**
ZUR OXIDATION VON ARSEN VERWENDBARE BAKTERIEN, VERFAHREN ZU IHRER SELEKTION UND IHRE VERWENDUNG ZUR AUFBEREITUNG VON ARSENHALTIGEN MEDIEN
BACTERIA USED FOR OXIDISING ARSENIC, METHOD FOR SELECTING SAME AND USES THEREOF FOR TREATING MEDIA CONTAINING ARSENIC

(30) Priorité: 03.10.2000 FR 0012579
(43) Date de publication de la demande: 14.01.2004
(73) Titulaire: B.R.G.M.-BUREAU DE RECHERCHES GEOLOGIQUES ET MINIERES, 75739 Paris Cedex 15 (FR)
(72) Inventeur: BATTAGLIA-BRUNET, Fabienne, F-45240 Marcilly en Villette (FR); MORIN, Dominique, F-45160 Olivet (FR); DICTOR, Marie-Christine, F-45000 Orléans (FR); BARANGER, Philippe, F-45750 Saint Pryve Saint Mesmin (FR)
(74) Mandataire: Puiroux, Guy
(86) Numéro de dépôt international: PCT/FR2001/003052
(87) Numéro de publication internationale: WO 2002/029007

(56) Documents cités:
- SANTINI, J.M. ET AL.: "A New Chemolithoautotrophic Arsenite-Oxidizing Bacterium Isolated from a Gold Mine: Phylogenetic, Physiological, and Preliminary Biochemical Studies" APPLIED AND ENVIRONMENTAL MICROBIOLOGY, vol. 66, no. 1, janvier 2000 (2000-01), pages 92-97, XP002171929
- WEEGER, W. ET AL.: "Oxidation of arsenite to arsenate by a bacterium isolated from an aquatic environment" BIOMETALS, vol. 12, no. 2, juin 1999 (1999-06), pages 141-149, XP000926523
- DATABASE WPI Section Ch, Week 198104 Derwent Publications Ltd., London, GB; Class D15, AN 1981-05147D XP002215223 & SU 734 274 A (AS KAZA MICROBIOL), 15 mai 1980 (1980-05-15)
- HAMBSCH, B. ET AL.: "Determination of Arsenic(III) for the Investigation of the Microbial Oxidation of Arsenic(III) to Arsenic(V)" ACTA HYDROCHIMICA ET HYDROBIOLOGICA, vol. 23, no. 4, 1995, pages 166-172, XP008008658
- BATTAGLIA-BRUNET, F. ET AL.: "An arsenic(III)-oxidizing bacterial population: selection, characterization, and performance in reactors" JOURNAL OF APPLIED MICROBIOLOGY, vol. 93, no. 4, 2002, pages 656-667, XP002215222

## Description

L'invention a pour objet des bactéries utilisables pour oxyder de l'arsenic sous forme d'arsénite As(III) en arséniate As(V). Elle vise également un procédé de sélection de telles bactéries, ainsi que leurs applications pour traiter des milieux renfermant de l'arsenic sous forme As(III).

La présence d'arsenic peut être observée dans des milieux naturels comme dans des matériaux ou résidus résultant d'activités humaines.

On le trouve ainsi dans des eaux souterraines, avec pour origine le fond géochimique. Il est également détectable, par exemple, dans les rejets d'eaux résiduaires, industrielles, dans les résidus de traitement de minerais, de combustion de charbon, de traitement de déchets, de dépôts de résidus industriels, ou lors de l'utilisation d'engrais.

Diverses voies d'élimination de l'arsenic ont été proposées, étant entendu que la valence sous laquelle se trouve l'arsenic à l'issue du traitement revêt une grande importance sur les rendements d'élimination.

Ainsi, la forme la mieux éliminée correspond à l'As(V) dans les traitements suivants: co-précipitation par les sels de fer ou par les sels d'aluminium, ou lors de traitement de décarbonatation à la chaux, osmose inverse.

Une autre voie d'élimination repose sur l'adsorption sur colonnes. Le matériau le plus utilisé est constitué par de l'alumine activée qui permet une bonne rétention de l'arsenic (III) et de l'arsenic (V). Cependant, la régénération de la colonne nécessite l'utilisation de soude caustique concentrée. Il s'ensuit que la récupération de l'As(III) est deux fois moins élevée que celle de l'As(V).

D'autres techniques, qui utilisent des résines échangeuses d'ions ou la nanofiltration, sont de mise en oeuvre complexe et s'accompagnent de coûts d'investissement et de maintenance élevés. De plus, la nanofiltration et l'osmose inverse entraînent la perte d'une partie de l'eau sous la forme d'un effluent concentré en arsenic.

Par ailleurs, une récente étude effectuée par le "Geological Survey of Finland" a montré que les trois méthodes couramment proposées pour l'élimination de l'arsenic dans les eaux issues de forages privés n'étaient pas efficaces à long terme. Ces méthodes correspondent à l'utilisation de cartouches à alumine activée, de cartouches à charbon actif, ou de résines échangeuses d'ions. Les meilleurs résultats ont été obtenus en oxydant l'As(III) en As(V) et en combinant ensuite deux méthodes d'épuration.

Comme souligné dans la littérature, l'efficacité de la plupart de ces traitements dépend de l'étape d'oxydation de l'As(III) en As(V).

L'oxydation de l'As(III) peut être réalisée par voie chimique, avec par exemple du chlore, du chlorure ferrique, du permanganate de potassium ou de l'ozone.

Les travaux des inventeurs dans ce domaine ont montré que, de manière inattendue, un tel processus d'oxydation pouvait être réalisé par catalyse biologique. Il est ainsi apparu qu'il était possible de faire l'économie des réactifs d'oxydation chimique en utilisant des bactéries sélectionnées pour leurs propriétés oxydantes spécifiques permettant d'oxyder As(III) en As(V).

La publication de SANTINI et al., A new chemolithoautotrophic Arsenite-Oxidizing baterium isolated from a Gold mine : phylogenetic, physiological and preliminary biochemical studies, APPLIED AND ENVIRONMENTAL MICROBIOLOGY, vol.66, no.1, January 2000 (2000-01), pages 92-97, divulgue des résultats similaires, obtenus avec une souche bactérienne autotrophe appartenant aux α-Protéobactéries.

L'invention a donc pour but de fournir de nouvelles bactéries capables d'oxyder As(III) en As(V) moins toxique, moins mobile et donc plus facile à éliminer par des traitements physico-chimiques conventionnels des milieux traités.

Selon un autre aspect, l'invention vise un procédé de sélection de telles bactéries, ainsi que les milieux de sélection en tant que nouveaux produits.

Selon encore un autre aspect, l'invention vise également l'utilisation des nouvelles bactéries pour le traitement de milieux renfermant de l'arsenic.

Les bactéries selon l'invention sont **caractérisées en ce qu**'il s'agit de bactéries isolées, autotrophes, aérobies, capables d'oxyder As(III) en As(V) en utilisant CO₂ comme seule source de carbone et As(III) comme seule source d'énergie et dont le génome présente au moins 80 % d'identité avec la souche déposée à la CNCM le 20 juin 2001 sous le numéro I-2687 et nommée CasO1.

Par ailleurs, au moins la partie de l'ADN des bactéries selon l'invention leur conférant lesdites propriétés, est capable de s'hybrider avec le génome de la souche déposée à la CNCM 28, rue du Dr Roux Paris, France le 20 juin 2001 sous le N° I-2687.

Il est en effet apparu que les bactéries selon l'invention issues d'un procédé de sélection tel que décrit ci-après relèvent majoritairement d'une seule espèce. Cette constatation résulte du séquençage de la région de l'ADN codant pour l'ARN ribosomal 16S, ci-après appelé ADNr 16S, après amplification de la région du génome de ces bactéries correspondant à la région 18-1492 du génome de *E*.*coli*, dont le numéro d'accession est cité par l'European Molecular Biology Library (EMBL) sous le numéro EMBL N°J01195, suivant la méthode fournie dans Brosius et al, 1981, Journal of Molecular Biology, 148 : 107-127. Cet ADNr 16S amplifié a été cloné dans *E*.*coli* puis séquencé.

Les séquences de l'ADNr 16S ainsi obtenues, réalisées sur 54 clones différents indiquent un taux de divergence supérieur à 5 % avec toutes les espèces bactériennes répertoriées, et une divergence inférieure à 3 % entre les différents clones sélectionnés analysés, comme expliqué en détail dans l'exemple 1 ci-après.

Les séquences ID n° 1 et n°2 représentent les séquences d'ADNr 16S issues des clones CO2 et C19 obtenus à partir de l'échantillon CasO1 ; la souche CasO1 contenant la séquence ID n°2 a été déposée à la CNCM le 20 juin 2001 sous le numéro I-2687.

Les bactéries de l'invention présentent en outre au moins une des caractéristiques suivantes :
- elles supportent jusqu'à 1 g.l⁻¹ au moins d'As(III) sans perdre leur aptitude à oxyder l'As(III),
- la vitesse d'oxydation de l'As(III) par ces bactéries en bioréacteur atteint au moins 4 mg.l⁻¹.h⁻¹,
- leur activité spécifique d'oxydation de l'As(III) atteint au moins 5x10⁻⁷ mg d'As(III) par bactérie et par heure. Avec une telle valeur d'activité spécifique, une suspension bactérienne contenant 10⁷ bactéries par ml est capable d'oxyder 5 mg d'As(III) par litre et par heure. Il s'ensuit que l'oxydation de l'As(III) par les bactéries, conformément à l'invention, ne nécessite qu'une très faible production de matière organique bactérienne. Par conséquent, les quantités de nutriments à apporter pour le développement des bactéries (azote, phosphore) sont également très faibles,
- elles sont capables de réduire la concentration résiduelle en As(III) à moins de 50 µg.l⁻¹.

Les bactéries selon l'invention et présentant les caractéristiques ci-dessus sont comprises dans un groupe constitué :
a) de la souche déposée à la CNCM le 20 juin 2001 sous le numéro I-2687 et nommée Cas01 ci-après ;
b) des souches dont le génome présente 80 % d'identité, de préférence 90 % d'identité avec la souche en a), l'identité étant entendue par une capacité de transcription et de traduction identiques.

L'invention vise également un procédé de sélection et d'isolement des bactéries décrites ci-dessus. Ce procédé est **caractérisé en ce qu**'il comprend la mise en contact d'un échantillon susceptible de renfermer de telles bactéries, en particulier prélevé sur un matériau contaminé par de l'arsenic, avec un milieu de sélection à raison de 50 à 500 mg.l⁻¹ d'arsenic sous forme d'arsenite, ne contenant pas de source de carbone ajoutée et ne fournissant que l'As(III) comme source d'énergie aux bactéries. L'échantillon et le milieu de sélection sont placés en conditions de culture stérile et aérobie. Le matériau contenant les bactéries que l'on souhaite sélectionner représente 5 à 20 % de la masse finale de culture.

La température d'incubation est comprise entre 20 et 35° C, et de préférence aux environs de 25° C, et le pH compris entre 5,5 et 6,5.

Le matériau contaminé provient par exemple d'un site minier ou industriel.

Au cours de la culture, des dosages d'AS(III) et d'AS(V) sont réalisés chaque jour. La culture est repiquée lorsque tout l'As(III) a été oxydé. Etant donné que le milieu de sélection est inoculé avec un échantillon susceptible de contenir des composés pouvant permettre la croissance de bactéries indésirables, il est préférable de réaliser des repiquages successifs afin de diluer les composés gênants. Des repiquages à environ 10 % sont avantageusement effectués à raison d'environ un repiquage toutes les 4 semaines pour les 3 premiers, puis chaque semaine pour les suivants.

Le milieu de sélection permet d'accroître progressivement la proportion de bactéries autotrophes oxydant l'As(III) en As(V) par rapport à la proportion des autres bactéries présentes ou indésirables. De plus, l'absence de source de carbone ajoutée assure également le développement des seules bactéries autotrophes pouvant se développer en utilisant le CO₂ atmosphérique comme source de carbone. Enfin, l'absence de source d'énergie autre que l'As(III) contribue à l'élimination des bactéries indésirables.

Un tel milieu de sélection est nouveau et, en tant que tel, entre également dans le champ de l'invention. Il est également utilisable pour la conservation des bactéries.

Pour la croissance des bactéries, outre l'As(III) à une concentration comprise entre 50 et 500 mg.l⁻¹, ce milieu renferme avantageusement en solution aqueuse des sels minéraux, et/ou des oligo-éléments et/ou des vitamines, étant entendu que ces nutriments ne doivent pas constituer un pool de composés oxydables qui pourrait être utilisé par les bactéries indésirables comme source d'énergie.

Les éléments minéraux comprennent avantageusement de l'azote, du phosphore, du potassium, du magnésium, du calcium, du sodium et un chlorure.

Un milieu approprié pour la sélection des bactéries selon l'invention est un milieu tamponné à pH sensiblement égal à 6 et contenant :
- de l'arsenite AS(III) à une concentration de 50 à 500 mg /l⁻¹ ;
- de l'ammonium ≤ 14 mg.l⁻¹, représentant 10 mg.l⁻¹ d'azote ;
- Des sels minéraux assurant une fonction de nutriment et/ou l'effet tampon mentionné ;

Ces sels minéraux suivants peuvent être :
- phosphates de di- et de mono- potassium, qui servent également de tampon de pH compris entre 0,05 et 2 g.l⁻¹
- sel de magnésium non biologiquement oxydable, comme le sulfate de magnésium entre 0,05 et 1 g.l⁻¹
- sel de calcium non biologiquement oxydable, comme le chlorure de calcium entre 0,05 et 1 g.l⁻¹
- sodium et chlorure, notamment sous forme de chlorure de sodium ou autre sel non biologiquement oxydable entre 0,05 et 1 g.l⁻¹
- métaux(oligo-éléments) : traces

On a constaté que la croissance des bactéries conformes à l'invention est corrélée au taux d'oxydation de l'As(III) comme montré dans les exemples.

Les bactéries susceptibles d'être sélectionnées par le procédé ci-dessus, en utilisant le milieu de sélection tel que défini, font également partie de l'invention.

L'invention concerne également un procédé de traitement de milieux renfermant de l'arsenic pour oxyder les arsénites en arséniates caractérisé par l'inoculation de ces milieux, dans un bioréacteur, avec une population bactérienne essentiellement constituée par des bactéries telles que définies et sélectionnées plus haut.

On opère avantageusement en milieu aqueux.

Une culture bactérienne est utilisée pour ensemencer un milieu à traiter de telle façon que la concentration finale de bactéries soit comprise entre 5.10⁵ et 5.10⁶ bactéries par ml, et de préférence environ 10⁶ bactéries par ml. En culture discontinue, ou batch, cela peut être atteint par l'inoculation du milieu à traiter avec 10 % volume à volume d'une culture bactérienne à confluence, à savoir environ 10⁷ bactéries par ml.

Dans un autre mode de réalisation, les bactéries peuvent être au moins en partie concentrées dans le bioréacteur par un support de fixation.

Lorsqu'on souhaite accélérer l'oxydation, on ajoute dans le milieu aqueux à traiter des sels minéraux servant d'éléments nutritifs et/ou on injecte de l'air lorsque la concentration en oxygène dans le milieu à traiter est limitante (ce qui dépend de la concentration initiale en As(III)).

Les éléments minéraux sont utilisés à raison d'environ 0,1 à 0,5 g.l⁻¹, notamment de 0,01 à 0,1 g.l⁻¹ du milieu inoculé et traité. Ces éléments sont choisis avantageusement parmi l'azote, le phosphore, le potassium et le magnésium.

Le procédé de traitement est avantageusement réalisé à un pH compris entre 5,5 et 6,5, de préférence aux environs de 6, et à une température comprise entre 20 et 35° C, de préférence entre 24 et 26° C.

Le traitement microbiologique est démarré en mode batch: l'As(III) contenu dans la solution à traiter est oxydé par les bactéries. On peut ensuite alimenter le bioréacteur par du milieu à traiter en mode continu. Ce milieu sera additionné le cas échéant d'éléments minéraux comme indiqué ci-dessus.

De manière avantageuse, on opère dans des conditions autorisant un contact homogène et efficace du milieu aqueux avec les bactéries.

A cet effet, on soumet par exemple le milieu réactionnel à agitation, en utilisant un bioréacteur équipé d'un dispositif d'agitation. En variante, on utilise un bioréacteur colonne à lit bactérien fixé.

La mise en oeuvre de ce procédé permet de réduire la toxicité du milieu, en conduisant à des concentrations résiduelles en As(III) inférieures à 50 µg.l⁻¹, et même à 10 µg.l⁻¹, comme requis par la directive européenne du 3 novembre 1998 en ce qui concerne la valeur limite de concentration en arsenic des eaux destinées à la consommation humaine. Les arséniates formés pourront être aisément éliminés grâce à leur mobilité plus faible que celle des arsénites. La solution récupérée à partir du bioréacteur, qui contient principalement de l'arsenic sous forme As(V) et une concentration résiduelle en As(III) inférieure à sa concentration initiale dans le milieu traité, est dirigée vers un dispositif d'élimination de l'As(V). Ce dispositif permet d'effectuer par exemple une co-précipitation par les sels de fer ou des sels d'aluminium ou une co-précipitation lors des traitements de décarbonatation à la chaux, une osmose inverse, une adsorption sur colonnes, ou tout autre procédé ou association de procédés conduisant à l'élimination de l'As(V).

Le procédé de l'invention est en particulier utilisable pour traiter des milieux riches en arsenic tels que les eaux destinées à la consommation humaine, la distribution collective ou les forages privés, ainsi que les effluents qu'ils soient miniers ou industriels, ou les eaux souterraines, notamment dans un procédé « in situ » de barrière réactive.

On décrira ci-après, à titre d'exemples non limitatifs, diverses formes d'exécution de la présente invention, en référence aux dessins annexés sur lesquels :
La figure 1 est un graphique représentant la variation de la concentration en As(V) en fonction du temps d'oxydation en "batch" de bactéries constituant la population Cas01.
La figure 2 est un graphique représentant la variation de la vitesse d'oxydation de l'As(III) en fonction du temps dans un bioréacteur fonctionnant en continu de bactéries constituant la population de Cas01.
La figure 3 est un graphique représentant, en fonction du temps, respectivement le temps de résidence des bactéries dans le réacteur et la vitesse d'oxydation de l'As(III).
La figure 4 est un graphique représentant la variation de la vitesse d'oxydation de l'As(III) en fonction du pH du milieu.
La figure 5 est un graphique représentant l'influence de la concentration en oxygène sur la vitesse d'oxydation de l'As(III) par les bactéries constituant la population bactérienne Cas01
La figure 6 est un graphique représentant l'influence de la température sur la vitesse d'oxydation de l'As(III) par les bactéries constituant la population Cas01.

### Exemple 1 Identification moléculaire d'une population bactérienne utilisée pour le traitement de l'arsenic

### · Techniques utilisées

### Principe de l'inventaire moléculaire

L'identification réalisée repose sur l'analyse de l'ARN 16S de la sous-unité ribosomale bactérienne, et de la séquence d'ADNr 16S dont il est transcrit, comme base de comparaison avec d'autres bactéries. On rappelle qu'il est admis à cet égard qu'une divergence de 3 % correspond à la distinction phénotypique entre deux espèces.

Après amplification, on procède au clonage dans *E*.*coli* de l'ADNr 16S de chaque bactérie présente dans l'échantillon, puis au séquençage, séquençage partiel de 500 pb dans tous les cas et séquençage complet de 1500 pb si la diversité est faible.

Les séquences obtenues sont d'abord comparées entre elles à l'aide d'un logiciel informatique pour distinguer chaque espèce présente sur la base du pourcentage de divergence.

Une interrogation de banques de données permet ensuite d'identifier les microorganismes les plus apparentés aux séquences représentatives de chaque espèce inventoriée.

GenBank et Ribosomal data Project ont été ainsi interrogées.

Les séquences représentatives des espèces inventoriées ont été une nouvelle fois comparées avec celles des microorganismes apparentés sur la base du pourcentage de divergence.

### Analyse SSCP

La technique SSCP (Single Strand Conformation Polymorphism) est basée sur la conformation secondaire qu'adopte un fragment d'ADN simple brin en conditions non-dénaturantes (grâce principalement à des attractions de types van der Waals) et qui est dépendante de sa structure primaire (séquence). Une région particulière de l'ADNr 16S (région V3 située environ entre la position 300 et 500) des micro-organismes présents est amplifiée, dénaturée pour séparer les brins d'ADN, puis analysée par électrophorèse sur gel d'électrophorèse en conditions non-dénaturantes.

Le profil d'analyse SSCP de la population globale permet de déterminer sa complexité et de différencier chaque micro-organisme présent grâce à la vitesse migratoire de son fragment d'ADNr.

L'assignation de chaque pic est ensuite réalisée par comparaison avec les profils d'analyse SSCP individuels de chaque clone représentatif des espèces recensées lors de l'inventaire moléculaire.

### Résultats

### - stabilisation des échantillons

A partir d'un échantillon de 15 ml, contenant environ 2x10⁹ bactéries/ml, on prélève 7 ml d'échantillon qu'on centrifuge aussitôt et le culot est repris dans 250 µl de guanidine thiocyanate et 37,5 µl de N-lauryl sarcosine 10%.

L'échantillon ainsi stabilisé est conservé à -20°C jusqu'à l'étape d'extraction de l'ADN total.

### - analyse et inventaire de la population bactérienne

L'ADN extrait de l'échantillon a été soumis à une réaction d'amplification de la région V3 des ADNr 16S présents et a été analysé selon la technique SSCP.

L'électrophorégramme SSCP indique qu'une espèce est essentiellement majoritaire puisqu'un seul pic apparaît sur le profil SSCP de la population globale.

L'analyse ultérieure des séquences des clones pour l'inventaire de la population bactérienne ainsi que les analyses SSCP individuelles de clones confirment cette observation.

Pour effectuer l'inventaire, une librairie de 54 clones a été constituée contenant chacun un fragment d'ADNr 16S bactérien.

Un criblage pour différencier les différentes populations présentes a été réalisé grâce à une digestion avec l'enzyme de restriction Hae III du fragment d'ADNr 16S de chaque clone suivie d'une électrophorèse sur gel d'agarose.

Les 54 clones analysés ont montré un profil de digestion identique. Ce résultat confirme la présence fortement majoritaire d'une seule espèce telle que seul l'ADNr de cette espèce est amplifié et est décelable.

La recherche sur les deux banques de données indique que la population majoritaire et exclusivement décelée par les techniques ci-dessus n'appartient d'un point de vue phylogénétique à aucune espèce déjà connue et répertoriée sur les banques de données. En effet, l'espèce la plus apparentée est *Thiomonas thermosulfata* (Proteobacteria ; sous-division bêta ; *Thiomonas*) avec un pourcentage de divergence relativement élevé puisque compris entre 7,9 et 7,3% dans le meilleur des cas (Tableau 1).

**Tableau 1. Clones dont l'ADNr 16S a été séquencé et divergence en % avec l'organisme le plus apparenté d'un point de vue phylogénétique qui est Thiomonas Thermosulfate.**

| Espèce apparentée | Clone | Divergence (%) avec l'espèce apparentée* |
|---|---|---|
| *Proteobacterium* | | |
| sous-division bêta | | |
| Thiomonas | | |
| *Thiomonas thermosulfata* | C02 | 7,3 |
| | C06 | 7,8 |
| | C10 | 7, 7 |
| | C16 | 7, 7 |
| | C19 | 7,7 |
| | C26 | 7,9 |
| | C30 | 7,7 |
| | C35 | 7,3 |
| | C37 | 7,3 |
| | C43 | 7,5 |
| | C45 | 7,3 |
| | C53 | 7,3 |
| | C56 | 7,8 |

| | | |
|---|---|---|
| * : une divergence de moins de 3% correspondant à la même espèce. | | |

### - analyse SSCP individuelle des clones et assignation

Une analyse SSCP a été réalisée sur 4 clones pris au hasard comptes tenu d'une part de la simplicité du profil SSCP de la population globale et d'autre part de la conservation parfaite de la région V3 de l'ADNr 16S des clones dont la séquence a été analysée.

Les profils SSCP des 4 clones analysés se superposent exactement sur le pic du profil SSCP de la population globale confirmant ainsi définitivement la détection exclusive d'une espèce de bactérie.

### - analyses complètes des séquences d'ADNr 16S

Compte tenu de la divergence importante des 500 premiers nucléotides de la séquence d'ADNr 16S de l'espèce détectée dans l'échantillon (3,7 à 7,9%) avec les séquences répertoriées dans les banques de données, une analyse de la séquence complète de l'ADNr 16S des 2 groupes de séquences majoritaires répertoriés, à savoir les groupes C6 et C2 a été réalisée. Bien que le pourcentage de divergence sur les 500 premiers nucléotides de l'ADNr 16S soit considéré comme représentatif de la divergence sur la totalité de la séquence, ces données complémentaires permettront ainsi de confirmer l'originalité phylogénétique de l'espèce détectée.

L'analyse complète de l'ADNr 16S des clones C02 (groupe C2) et C19 (groupe C6) montre d'une part que les séquences sont divergentes entre elles de seulement 0,1% et d'autre part qu'après comparaison avec les banques de données ces séquences appartiennent bien à une espèce différente de celles actuellement connues et répertoriées (tableau 2).

Tableau 2. Divergence en % des ADNr 16S des clones C02 et C19 avec ceux des micro-organismes les plus apparentés**.

| Espèce apparentée | Clone | Clone |
|---|---|---|
| | C02 | C19 |
| *Thiomonas thermosulfata* | 6,2 | 6,3 |
| Bacterie dégradant l'acide déhydroabiétique | 7,7 | 7,8 |
| *Leptothrix discophora* | 7,5 | 7,7 |
| *Leptothrix mobilis* | 7,9 | 8,1 |

| | | |
|---|---|---|
| ** : une divergence de moins de 3% correspond à la même espèce. Les séquences d'ADNr 16S correspondant aux clones CO2 et C19 sont fournies respectivement dans les séquences ID n°1 et n°2. | | |

GenBank indique que la séquence la plus proche répertoriée est celle d'une bêta Protéobactérie de la famille des Comamonadaceae dénommée dehydroabietic acid-degrading bacterium DhA-7.

La banque Ribosomal Data Project indique que les séquences les plus apparentées sont celles de *Leptothrix discophora* str. SS-1 et de *Leptothrix mobilis* str. Feox-1 également des Comamonadaceae.

L'étude d'inventaire moléculaire réalisée sur la population de l'échantillon CAs01 montre donc qu'une seule population a été détectée et les micro-organismes qui sont les plus apparentés d'un point de vue phylogénétique sont les bêta Protéobactéries *Thiomonas thermosulfata*, Dehydroabietic acid-degrading bacterium DhA-71, *Leptothrix discophora* str. SS-1 et *Leptothrix mobilis* str. Feox-1.

La détection d'une seule espèce de micro-organisme dans l'échantillon CAs01 indique que le milieu de l'échantillon est extrêmement sélectif et proche des conditions d'une culture pure (aucune autre espèce contaminante n'a été détectée parmi les 54 clones analysés).

Les travaux de comparaisons montrent que la population de CAs01 est nouvelle puisqu' aucune bactérie connue et répertoriée dans les différentes banques de données ne possède une divergence au moins inférieure à 6,2% avec l'organisme inventorié.

### Exemple 2 : Composition d'un milieu de sélection

### I - Préparation du milieu de culture

### • Milieu de base

Le milieu de base est composé d'une solution A et d'une solution B.

### Solution A :

| | |
|---|---|
| KH₂PO₄ | 0,5 g |
| K₂HPO₄ | 0,5 g |
| NaCl | 0,5 g |
| (NH₄)₂SO₄ | 0,05 g |
| Eau déminéralisée | 500 ml |

La solution A est préparée dans un flacon en verre de 1 litre. Le pH de la solution est ajusté à pH 6.

### Solution B :

| | |
|---|---|
| MgSO₄, 7 H₂O | 0,1 g |
| CaCl₂, 2 H₂O | 0,1 g |
| Eau déminéralisée | 500 ml |

La solution B est préparée dans un flacon en verre de 500 ml.

Les solutions A et B sont stérilisées par autoclavage pendant 20 minutes à 120°C.

### • Solution d'As(III)

Une solution aqueuse de As₂O₃ à 13 g.l⁻¹, correspondant à une solution à 10 g.l⁻¹ d'As(III) est préparée de la façon suivante. L'As₂O₃ étant peu soluble dans l'eau, il est nécessaire de procéder à une dissolution à chaud en milieu basique (0,55 g de NaOH dans 100 ml d'eau à ébullition).

La solution d'As(III) est placée dans un flacon stérile de 1 litre.

### • Solution d'oligo-éléments

| | |
|---|---|
| HCl en solution à 25% | 6,5 ml |
| FeCl2, 4 H₂O | 1,5 g |
| H₃BO₃ | 60 mg |
| MnSO₄, H₂O | 117 mg |
| CoCl₂, 6 H₂O | 25 mg |
| ZnCl₂ | 70 mg |
| NiCl₂, 6 H₂O | 25 mg |
| CuCl₂, 2 H₂O | 15 mg |
| Na₂MoO₄, 2 H₂O | 25 mg |
| Eau déminéralisée | 1000 ml |

La solution d'oligo-éléments est stérilisée par filtration à 0,45 µm dans un flacon en verre stérile.

### • Solution de vitamines

| | |
|---|---|
| Acide p-amino-benzoïque | 10 mg |
| Biotine | 10 mg |
| Acide folique | 4 mg |
| Pyridoxine-HCl | 2 mg |
| Riboflavine | 10 mg |
| Thiamine | 10 mg |
| Acide nicotinique | 10 mg |
| Acide pantothénique | 10 mg |
| B12 | 0,2 mg |
| Vitamine Eau déminéralisée | 1000 ml |

La solution de vitamines est stérilisée par filtration à 0, 45 µm.

### II - Préparation du milieu complet

En conditions stériles, il est nécessaire de compléter le milieu avec les solutions suivantes.

| | |
|---|---|
| Milieu de base | 1000 ml |
| Solution d'oligo-éléments | 1 ml |
| Solution de vitamines | 10 ml |
| Solution d'As(III) | 10 ml |

Le milieu est stocké à température ambiante.

### Exemple 3 : Procédé d'oxydation de l'As(III) en batch par les bactéries constituant la population Cas01.

La population bactérienne Cas01, sélectionnée avec le milieu de l'exemple 2, est cultivée dans un milieu contenant 100 mg.l⁻¹ d'As(III). Les expérimentations sont réalisées dans des erlenmeyers de 500 ml en verre stérile, placés sur une table d'agitation animée d'un mouvement de va et vient, thermostatée à une température de 25°C. Ces erlenmeyers contenant 250 ml de milieu de l'exemple 2, sont ensemencés avec 10% de suspension bactérienne issue de Cas01 à 10⁷ bactéries ml⁻¹. Sans phase d'adaptation préalable, cette population bactérienne est utilisée pour inoculer des milieux contenant entre 100 et 1000 mg.l⁻¹ d'As(III).

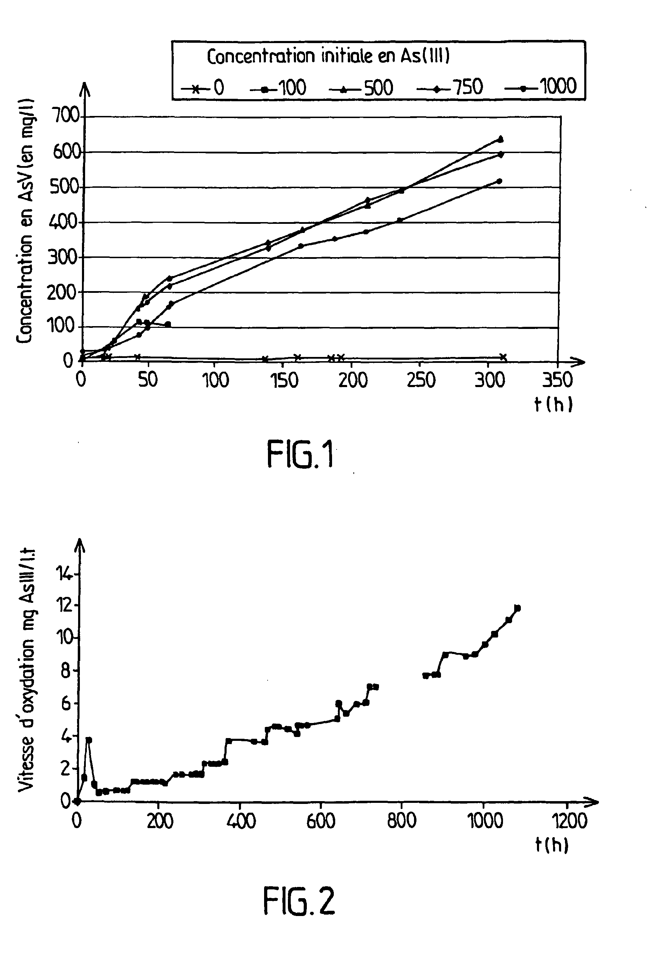

Les cinétiques d'oxydation de l'As(III) en As (V) ainsi obtenues sont représentées par le graphe de la figure 1, où l'axe des ordonnées représente la concentration en As (V) généré par l'oxydation de l'As(III), en mg.l⁻¹, et l'axe des abscisses représente le temps en heures.

Lorsque le milieu est ajusté initialement à pH 6, l'oxydation de As(III) en As(V) est corrélée à une chute de pH.

Cet exemple montre que la population bactérienne CAs01 conserve sa capacité à oxyder l'As(III) lorsqu'elle est placée dans une solution contenant 1000 mg.l⁻¹ d'As(III), soit dix fois la concentration en As(III) présente dans son milieu de culture habituel.

### Exemple 4 Procédé d'oxydation de l'As(III) en continu par les bactéries constituant la population Cas01.

La population bactérienne Cas01, sélectionnée avec le milieu de l'exemple 2, a été utilisée pour inoculer un réacteur muni d'un axe d'agitation équipé d'une hélice, et d'un système d'aération. Ce bioréacteur a été rempli avec une solution contenant 100 mg.l⁻¹ d"As(III). Après la phase batch, au cours de laquelle les bactéries ont oxydé entièrement l'As(III) en As (V), le bioréacteur a été alimenté en continu avec une solution contenant 100 mg.l⁻¹ d'As(III). Le débit d'alimentation en solution chargée en arsenic a été progressivement accru. Le temps de résidence de la solution dans le bioréacteur a ainsi été réduit de 150 heures à 10 heures. Le pourcentage d'oxydation de l'arsenite par le bioréacteur a toujours été supérieur à 99 %. L'augmentation de la vitesse d'oxydation de l'As(III) dans le bioréacteur est représentée par le graphe de la figure 2, où l'axe des ordonnées représente la vitesse d'oxydation de l'As(III) en mg.l⁻¹.h⁻¹, et l'axe des abscisses représente le temps en heures.

Il montre que la vitesse d'oxydation peut être de 12 mg AS(III).l⁻¹.h⁻¹ alors qu'en batch cette vitesse est au maximum de 4 mg.l⁻¹.h⁻¹.

### Exemple 5 :Procédé d'oxydation de l'As(III) en continu dans un bioréacteur à lit fixé par les bactéries constituant la population Cas01

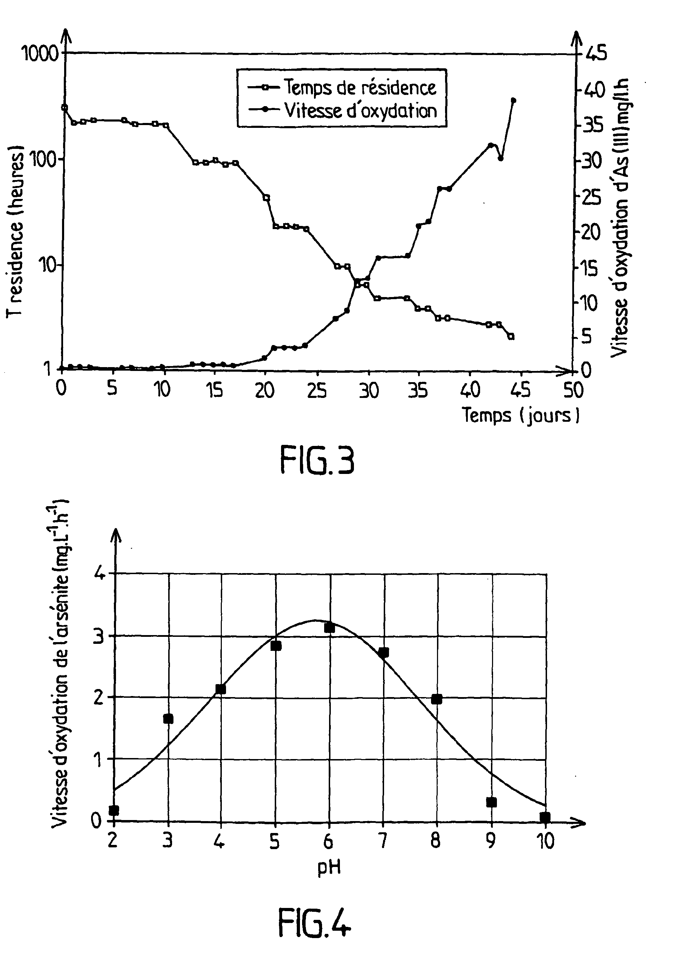

La population bactérienne a été utilisée pour inoculer un réacteur colonne à lit fixé, contenant de la pouzzolane en tant que support pour la fixation des bactéries. Une injection d'air à la base de la colonne a permis d'assurer l'oxygénation du système. Le bioréacteur a été alimenté en continu avec un milieu de culture contenant 100 mg.l⁻¹ d'As(III). Les nutriments ont été apportés dans les proportions suivantes : 50 mg.l⁻¹ de (NH₄)₂SO₄, 50 mg.l⁻¹ de MgSO₄, 7 H₂O, 50 mg.l⁻¹ de K₂HPO₄, 50 mg.l⁻¹ de KH₂PO₄, 50 mg.l⁻¹ de NaCl. Il n'a pas été nécessaire d'ajouter une source de calcium dans ce milieu, car celui-ci a été préparé avec de l'eau de ville contenant déjà du calcium. Le traitement a été réalisé dans des conditions non stériles. Le débit d'alimentation en solution chargée en arsenic a été progressivement augmenté. Le temps pendant lequel la solution a résidé dans le bioréacteur a ainsi été réduit de 300 heures à 2 heures. Le pourcentage d'oxydation de l'arsenic (III) par le bioréacteur a toujours été supérieur à 95%. L'augmentation de la vitesse d'oxydation de l'As(III) dans le bioréacteur et la diminution du temps de résidence ont été représentées sur le graphique de la figure 3, sur lequel l'axe des ordonnées de gauche représente le temps de résidence en heures, l'axe des ordonnées de droite représente la vitesse d'oxydation de l'AS(III) en mg.l⁻¹.h⁻¹, et l'axe des abscisses représente le temps en jours. Dans de telles conditions, on a pu obtenir une vitesse d'oxydation au moins égale à 38 mg.l⁻¹.h⁻¹.

### Exemple 6 : Influence du pH sur la vitesse d'oxydation de l'As(III)par les bactéries constituant la population Cas01

La population bactérienne Cas01 a été utilisée pour inoculer des réacteurs dont le volume utile était de 1 litre, et qui étaient munis de moyens d'agitation constitués d'un axe équipé d'une hélice, ainsi que d'un système d'aération. Ces réacteurs ont été remplis avec un litre de milieu minéral contenant 100 mg.l⁻¹ d'As(III). Le pH initial du milieu dans ces réacteurs a été ajusté à différentes valeurs comprises entre 2 et 10 ainsi que représenté sur la figure 4. Les réacteurs ont été inoculés avec 100 ml de culture Cas01. Les cultures ont été réalisées en condition dite "batch", ce qui signifie que le milieu n'a pas été renouvelé. Des prélèvements fréquents ont été effectués dans les réacteurs et ont permis de suivre le processus d'oxydation de l'As(III) et de calculer une vitesse d'oxydation de celui-ci pour chaque condition de pH.

L'influence du pH sur la vitesse d'oxydation de l'As(III) par la population bactérienne Cas01 est ainsi illustrée sur la figure 4, et on constate sur celle-ci que cette vitesse est maximale entre pH 5 et pH 7. On notera également que la vitesse est encore élevée entre pH 3 et pH 8, mais devient faible pour des valeurs de pH inférieures à 3 et supérieures à 8.

### Exemple 7 : Influence de la concentration en oxygène sur la vitesse d'oxydation de l'As(III) par les bactéries constituant la population bactérienne Cas01.

Cette population bactérienne Cas01 a été utilisée pour inoculer un réacteur d'un volume utile de 10 litres, muni de moyens d'agitation constitués d'un axe équipé d'une hélice et d'un système permettant d'injecter de l'azote ou de l'air dans le milieu de culture. Le réacteur a été rempli avec 10 litres de milieu minéral contenant 100 mg.l⁻¹ d'As(III). Le pH initial du milieu dans ce réacteur a été ajusté à 6. L'air présent dans le réacteur a été entièrement chassé par un flux d'azote, puis la concentration en oxygène dans le milieu a été ajustée à différentes valeurs comprises entre 0 et 0,3 mMole.l⁻¹ par injection de faibles volumes d'air. Le réacteur a été inoculé avec un litre de culture Cas01. Les essais successifs à différentes concentrations en oxygène ont été réalisés ainsi que précédemment dans des conditions de "batch". Des prélèvements fréquents effectués dans le réacteur ont permis de suivre le processus d'oxydation de l'As(III) et de calculer une vitesse d'oxydation de celui-ci pour chaque concentration en oxygène.

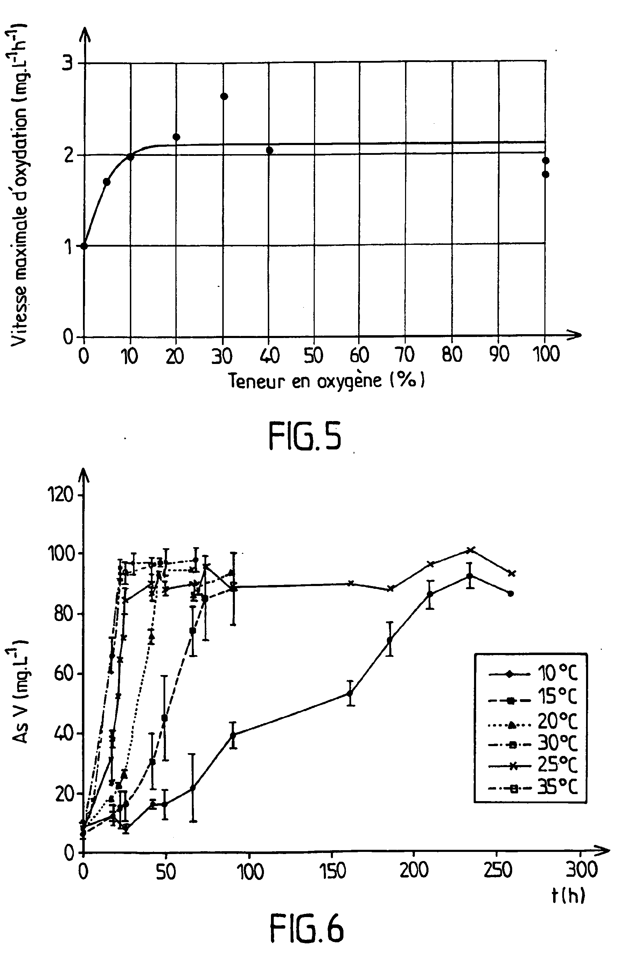

L'influence de la concentration en oxygène sur la vitesse d'oxydation de l'As(III) par la population bactérienne Cas01 est illustrée sur la figure 5. Cette vitesse maximale d'oxydation a été obtenue dès que la concentration initiale en oxygène dissous a atteint 0,05 mMole.l⁻¹, soit environ 10% de la concentration lorsque le milieu liquide a été saturé par l'oxygène. Dans ces conditions, on a constaté que le besoin en oxygène de la population bactérienne Cas01 pour oxyder l'As(III) est faible.

### Exemple 8 : Influence de la température sur la vitesse d'oxydation de l'As(III) par les bactéries constituant la population Cas01

Ainsi que précédemment, on a utilisé la population bactérienne Cas01 pour inoculer des réacteurs dont le volume utile était cette fois-ci de un litre, et qui était muni de moyens d'agitation et d'un système d'aération. Les réacteurs ont été remplis avec un litre d'un milieu minéral contenant 100 mg.l⁻¹ d'As(III). Le pH initial du milieu dans ces réacteurs a été ajusté à 6. Les réacteurs étaient des réacteurs thermostatés, par circulation d'eau provenant d'un cryo-thermostat dans une double enveloppe. La température a été maintenue à différentes valeurs fixes comprises entre 10°C et 35°C. Les réacteurs ont été inoculés avec 100 ml de culture Cas01. Ces cultures ont été réalisées en condition "batch" ainsi que précédemment. Des prélèvements fréquents effectués dans les réacteurs ont permis de suivre le processus d'oxydation et de calculer une vitesse d'oxydation de l'As(III) pour chaque condition de température.

L'influence de la température sur l'oxydation de l'As(III) est illustrée sur la figure 6. On constate sur celle-ci que la vitesse d'oxydation de l'As(III) par la population bactérienne Cas01 est maximale à partir d'une température de 25°C, et que cette vitesse d'oxydation est encore élevée à des températures de 20°C et même de 15°C, mais qu'elle est sensiblement plus faible à une température de 10°C.

## Revendications

1. Bactéries autotrophes, aérobies, aptes à oxyder l'arsenic sous forme d'arsenite As(III) en arseniate As(V) en utilisant le CO2 comme seule source de carbone et l'As(III) comme seule source d'énergie, **caractérisées en ce qu'**elles sont comprises dans un groupe constitué :
a) de la souche déposée à la CNCM le 20 juin 2001 sous le numéro I-2687 et nommée Cas01 ;
b) des souches dont le génome présente 80 % d'identité, de préférence 90 % d'identité, avec la souche en a) ;
**et en ce qu**'elles présentent en outre au moins une des caractéristiques suivantes :
- elles supportent jusqu'à 1 g.l⁻¹ au moins d'As(III) atteint sans perdre leur aptitude à oxyder l'As(III),
- la vitesse d'oxydation de l'As(III) par ces bactéries en bioréacteur atteint au moins 4 mg.l⁻¹.h⁻¹,
- leur activité spécifique d'oxydation de l'As(III) atteint au moins 5x10⁻⁷ mg d'As(III) par bactérie et par heure,
- elles sont capables de réduire la concentration résiduelle en As(III) à moins de 50 µg.l⁻¹.

2. Procédé de sélection de bactéries selon la revendication 1, **caractérisé en ce que** ledit procédé comprend :
a) une mise en contact d'un échantillon susceptible de renfermer de telles bactéries avec un milieu de sélection contenant de 50 à 500 mg.l⁻¹ d'arsenite As(III) et des sels minéraux ;
b) la mise en culture en conditions stériles de l'échantillon et du milieu de sélection, dans des conditions de pH comprises entre 5,5 et 6,5 et de température comprise entre 20°C et 35°C ;
c) la sélection des bactéries aptes à oxyder l'As(III) en As(V) le cas échéant par des repiquages successifs dans le milieu de sélection.

3. Procédé selon la revendication 2 dans lequel le matériau contenant les bactéries que l'on souhaite sélectionner représente 5 à 20 % de la masse finale de culture.

4. Procédé selon les revendications 2 ou 3 dans lequel le milieu de sélection contient :
- de l'arsenite : de 50 à 500 mg.l⁻¹,
- de l'ammonium : ≤ 14 mg.l⁻¹,
- des sels minéraux aptes à assurer une fonction de nutriment et/ou un effet tampon dudit milieu.

5. Procédé selon l'une quelconque des revendications 2 à 4, **caractérisé en ce que** le milieu de sélection comprend les composés suivants:
- ammonium ≤ 14 mg.l⁻¹, soit 10 mg.l⁻¹ d'azote ;
- phosphates de di- et de mono- potassium, qui servent également de tampon de pH compris entre 0,05 et 2 g.l⁻¹ ;
- sel de magnésium non biologiquement oxydable, comme le sulfate de magnésium entre 0,05 et 1 g.l⁻¹
- sel de calcium non biologiquement oxydable, comme le chlorure de calcium entre 0,05 et 1 g.l⁻¹;
- sodium et chlorure, notamment sous forme de chlorure de sodium ou autre sel non biologiquement oxydable entre 0,05 et 1 g.l⁻¹ ;
- métaux(oligo-éléments) : traces.

6. Procédé de traitement de milieux renfermant de l'arsenic pour oxyder les arsénites en arséniates, **caractérisé par** l'inoculation de ces milieux dans un bioréacteur avec une population bactérienne essentiellement constituée par des bactéries selon la revendications 1 et/ou sélectionnées par un procédé selon l'une quelconque des revendications 2 à 5.

7. Procédé selon la revendication 6, **caractérisé en ce que** les bactéries sont inoculées à raison de 5.10⁵ à 5.10⁶ bactéries par ml de milieu final, de préférence de l'ordre de 10⁶ bactéries/ml.

8. Procédé selon l'une des revendications 6 ou 7 **caractérisé en ce que** les bactéries sont au moins en partie concentrées dans le bioréacteur par un support de fixation.

9. Procédé selon l'une quelconque des revendications 6 à 8, **caractérisé en ce que,** pour accélérer l'oxydation, on ajoute dans le milieu aqueux à traiter des éléments minéraux servant d'éléments nutritifs et/ou on injecte de l'air.

10. Procédé selon la revendication 9 dans lequel les éléments minéraux sont :
- ammonium ≤ 14 mg.l⁻¹, soit 10 mg.l⁻¹ d'azote ;
- phosphates de di- et de mono- potassium, qui servent également de tampon de pH compris entre 0,05 et 2 g.l⁻¹
- sel de magnésium non biologiquement oxydable, comme le sulfate de magnésium entre 0,05 et 1 g.l⁻¹
- sel de calcium non biologiquement oxydable, comme le chlorure de calcium entre 0,05 et 1 g.l⁻¹
- sodium et chlorure, notamment sous forme de chlorure de sodium ou autre sel non biologiquement oxydable entre 0,05 et 1 g.l⁻¹
- métaux(oligo-éléments) : traces

11. Procédé selon l'une quelconque des revendications 6 à 10, **caractérisé en ce que** le traitement microbiologique est effectué en mode batch ou en continu.

12. Procédé selon l'une quelconque des revendications 6 à 11, **caractérisé en ce que** le milieu traité est pris parmi : les eaux destinées à la consommation humaine, distribution collective ou forages privés, les effluents miniers ou industriels, les eaux souterraines.

## Claims

1. Autotrophic aerobic bacteria capable of oxidizing arsenic in the form of arsenite As(III) into arsenate As(V) by using CO₂ as the only source of carbon and As(III) as the only source of energy, **characterized in that** they are included in a group comprised of:
a) the strain filed with the CNCM on June 20, 2001 under number I-2687 and named Cas01;
b) strains whose genome exhibits 80% identity, preferably 90% identity, with the strain in a);
and **in that** they further exhibit at least one of the following characteristics:
- they support up to at least 1 g/l of As (III) without losing their ability to oxidize As(III),
- the As(III) oxidation rate of these bacteria in a bioreactor reaches at least 4 mg/l/h,
- their specific As(III) oxidation activity reaches at least 5x10⁻⁷ mg of As(III) per bacterium per hour,
- they are able to reduce the residual concentration of As(III) to less than 50 µg/l.

2. The method for selecting bacteria of claim 1, **characterized in that** said method comprises:
a) placing a sample likely to contain such bacteria in contact with a selection medium containing from 50 mg/l to 500 mg/l of arsenite As(III) and mineral salts;
b) culturing the sample and the selection medium in sterile conditions at a pH between 5.5 and 6.5 and at a temperature between 20°C and 35°C;
c) selecting bacteria capable of oxidizing As(III) into As(V), if necessary by successive stabs in the selection medium.

3. The method of claim 2, wherein the material containing the bacteria which is sought to be selected represents 5% to 20% of the final mass of the culture.

4. The method of claim 2 or 3, wherein the selection medium contains:
- arsenite: from 50 mg/l to 500 mg/l,
- ammonium: ≤14 mg/l,
- mineral salts capable of providing a nutrient function and/or a buffer effect for said medium.

5. The method of any of claims 2 to 4, **characterized in that** the selection medium contains the following compounds:
- ammonium ≤14 mg/l, or 10 mg/l of nitrogen;
- di- and mono-potassium phosphates, which also act to buffer the pH, between 0.05 g/l and 2 g/l;
- non-biologically oxidizable magnesium salt, such as magnesium sulfate, between 0.05 g/l and 1 g/l;
- non-biologically oxidizable calcium salt, such as calcium chloride, between 0.05 g/l and 1 g/l;
- sodium and chloride, notably in the form of sodium chloride or another non-biologically oxidizable salt, between 0.05 g/l and 1 g/l;
- metals (trace elements): traces.

6. A method for treating media containing arsenic to oxidize arsenites into arsenates, **characterized by** the inoculation of these media in a bioreactor with a bacterial population essentially comprised of the bacteria of claim 1 and/or selected by the method of any of claims 2 to 5.

7. The method of claim 6, **characterized in that** the bacteria are inoculated at a concentration of 5 x 10⁵ to 5 x 10⁶ bacteria per ml of final medium, preferably on the order of 10⁶ bacteria/ml.

8. The method of one of claims 6 or 7, **characterized in that** the bacteria are at least partly concentrated in the bioreactor by a fixation support.

9. The method of any of claims 6 to 8, **characterized in that**, to accelerate oxidation, mineral elements serving as nutrient elements are added and/or air is injected in the aqueous medium to be treated.

10. The method of claim 9, wherein the mineral elements are:
- ammonium ≤14 mg/l, or 10 mg/l of nitrogen;
- di- and mono-potassium phosphates, which also act to buffer the pH, between 0.05 g/l and 2 g/l;
- non-biologically oxidizable magnesium salt, such as magnesium sulfate, between 0.05 g/l and 1 g/l;
- non-biologically oxidizable calcium salt, such as calcium chloride, between 0.05 g/l and 1 g/l;
- sodium and chloride, notably in the form of sodium chloride or another non-biologically oxidizable salt, between 0.05 g/l and 1 g/l;
- metals (trace elements): traces.

11. The method of any of claims 6 to 10, **characterized in that** the microbiological treatment is carried out in batch mode or continuously.

12. The method of any of claims 6 to 11, **characterized in that** the treated medium is selected among: water intended for human consumption, public distribution or private wells, mining or industrial wastewater, ground water.

## Patentansprüche

1. Autotrophe, aerobe Bakterien, die fähig sind, Arsen in Form von Arsenit As (III) in Arsenat As (V) zu oxidieren, und zwar unter Verwendung des CO₂ als einzige Kohlenstoffquelle und des As(III) als einzige Energiequelle, **dadurch gekennzeichnet, dass** sie in einer Gruppe enthalten sind, die gebildet ist:
a) durch den Stamm, der bei der CNCM am 20. Juni 2001 unter der Nummer I-2687 hinterlegt wurde und als Cas01 bezeichnet ist;
b) durch Stämme, deren Genom 80 % Identität, vorzugsweise 90 % Identität, mit dem Stamm unter a) aufweist;
**und dadurch, dass** sie außerdem mindestens eine der folgenden Eigenschaften aufweisen:
- sie mindestens bis zu 1 g • l⁻¹ von As (III) vertragen, wobei dieser Wert erreicht wird, ohne dass sie ihre Fähigkeit zum Oxidieren von As (III) verlieren,
- die Geschwindigkeit der Oxidation von As (III) durch diese Bakterien im Bioreaktor mindestens 4 mg • l⁻¹ • h⁻¹ erreicht,
- ihre spezifische Aktivität zur Oxidation von As (III) mindestens 5 x 10⁻⁷ mg As (III) pro Bakterie und pro Stunde erreicht,
- sie fähig sind, die Restkonzentration von As (III) auf weniger als 50 µg • l⁻¹ zu reduzieren.

2. Verfahren zur Selektion von Bakterien nach Anspruch 1, **dadurch gekennzeichnet, dass** das Verfahren umfasst:
a) In-Kontakt-Bringen einer Probe, die fähig ist, derartige Bakterien zu enthalten, mit einem Selektionsmedium, das 50 bis 500 mg • l⁻¹ Arsenit As (III) und Mineralsalze enthält;
b) In-Kultur-Bringen unter sterilen Bedingungen der Probe und des Selektionsmediums, und zwar unter Bedingungen eines pH-Wertes zwischen 5,5 und 6,5 und einer Temperatur zwischen 20 °C und 35 °C;
c) Selektieren von Bakterien, die fähig sind, As (III) zu As (V) zu oxidieren, gegebenenfalls durch sukzessive erneute Pikiervorgänge im Selektionsmedium.

3. Verfahren nach Anspruch 2, bei dem das Material, das die Bakterien enthält, die man selektionieren möchte, 5 bis 20 % der Endmasse der Kultur darstellt.

4. Verfahren nach den Ansprüchen 2 oder 3, bei dem das Selektionsmedium enthält:
- Arsenit: 50 bis 500 mg • l⁻¹,
- Ammonium: ≤ 14 mg • l⁻¹,
- Mineralsalze, die fähig sind, für eine Ernährungsfunktion und/oder einen Puffereffekt des Mediums zu sorgen.

5. Verfahren nach einem der Ansprüche 2 bis 4, **dadurch gekennzeichnet, dass** das Selektionsmedium folgende Verbindungen enthält:
- Ammonium ≤ 14 mg • l⁻¹, das bedeutet 10 mg • l⁻¹ Stickstoff;
- Bi- und Mono-Kaliumphosphate, die ebenfalls als pH-Puffer dienen, zwischen 0,05 und 2 g • l⁻¹;
- nicht biologisch oxidierbares Magnesiumsalz, wie beispielsweise Magnesiumsulfat, zwischen 0,05 und 1 g • l⁻¹;
- nicht biologisch oxidierbares Calciumsalz, wie beispielsweise Calciumchlorid, zwischen 0,05 und 1 g • l⁻¹;
- Natrium und Chlorid, insbesondere in Form von Natriumchlorid oder einem anderen nicht biologisch oxidierbaren Salz, zwischen 0,05 und 1 g • l⁻¹;
- Metalle (Spurenelemente): Spuren.

6. Verfahren zur Behandlung von arsenhaltigen Medien zum Oxidieren von Arseniten in Arsenate, **gekennzeichnet durch** die Inokulation dieser Medien in einem Bioreaktor mit einer bakteriellen Population, die im Wesentlichen **durch** Bakterien nach Anspruch 1 und/oder **durch** solche, die durch ein Verfahren gemäß einem der Ansprüche 2 bis 5 selektioniert wurden, gebildet ist.

7. Verfahren nach Anspruch 6, **dadurch gekennzeichnet, dass** die Bakterien im Verhältnis von 5 • 10⁵ bis 5 • 10⁶ Bakterien pro ml des Endmediums, vorzugsweise in der Größenordnung von 10⁶ Bakterien/ml inokuliert werden.

8. Verfahren nach einem der Ansprüche 6 oder 7, **dadurch gekennzeichnet, dass** die Bakterien zumindest teilweise im Bioreaktor mittels eines Fixierträgers konzentriert sind.

9. Verfahren nach einem der Ansprüche 6 bis 8, **dadurch gekennzeichnet, dass,** um die Oxidation zu beschleunigen, dem zu behandelnden wässrigen Medium Mineralstoffe zugesetzt werden, die als Nährstoffe dienen, und/oder Luft zugeführt wird.

10. Verfahren nach Anspruch 9, bei dem die Mineralstoffe sind:
- Ammonium ≤ 14 mg • l⁻¹, das bedeutet 10 mg • l⁻¹ Stickstoff;
- Bi- und Mono-Kaliumphosphate, die ebenfalls als pH-Puffer dienen, zwischen 0,05 und 2 g • l⁻¹;
- nicht biologisch oxidierbares Magnesiumsalz, wie beispielsweise Magnesiumsulfat, zwischen 0,05 und 1 g • l⁻¹;
- nicht biologisch oxidierbares Calciumsalz, wie beispielsweise Calciumchlorid, zwischen 0,05 und 1 g • l⁻¹;
- Natrium und Chlorid, insbesondere in Form von Natriumchlorid oder einem anderen nicht biologisch oxidierbaren Salz, zwischen 0,05 und 1 g • l⁻¹;
- Metalle (Spurenelemente): Spuren.

11. Verfahren nach einem der Ansprüche 6 bis 10, **dadurch gekennzeichnet, dass** das mikrobiologische Verfahren im chargenweisen Modus oder kontinuierlich durchgeführt wird.

12. Verfahren nach einem der Ansprüche 6 bis 11, **dadurch gekennzeichnet, dass** das behandelte Medium unter den folgenden gewählt ist: Wasser für den menschlichen Verbrauch, Gemeinschaftsversorgung oder private Brunnen, Abwässer aus Bergbau oder Industrie, Grundwasser.
